# EUROPÄISCHE PATENTSCHRIFT

(11) **EP 2 492 202 B1**
(45) Veröffentlichungstag und Bekanntmachung des Hinweises auf die Patenterteilung: **17.12.2014**
(21) Anmeldenummer: 12169537.3
(22) Anmeldetag: 18.08.2009
(51) Int. Cl.: B65B 55/08, A61L 2/08, A61L 2/10, A61L 2/24, B67C 7/00

(54) **Elektronenstrahlsterilisation für Behältnisse**
Electron beam sterilisation for containers
Stérilisation par rayonnement d'électrons pour récipients

(30) Priorität: 30.08.2008 DE 102008045187
(43) Veröffentlichungstag der Anmeldung: 29.08.2012
(62) Teilanmeldung aus: 09168082.7
(73) Patentinhaber: Krones AG, 93073 Neutraubling (DE)
(72) Erfinder: Krüger, Jochen, 93095 Hagelstadt (DE); Humele, Heinz, 93107 Thalmassing (DE)
(74) Vertreter: Bittner, Bernhard

(56) Entgegenhaltungen:
- EP-A- 1 120 121
- EP-A- 1 944 044
- WO-A-97/07024
- WO-A-99/39751
- US-A1- 2005 135 965
- US-A1- 2005 173 020
- US-A1- 2006 192 140
- US-A1- 2007 283 667

## Beschreibung

Die vorliegende Erfindung bezieht sich auf eine Vorrichtung und ein Verfahren zum Sterilisieren von Behältnissen und insbesondere ein Verfahren und eine Vorrichtung zum Sterilisieren von Behältnissen mittels Elektronenstrahlen.

Im Bereich der Getränke herstellenden Industrie ist es bekannt, Kunststoffbehältnisse zunächst zu formen und anschließend mit einem Getränk zu befüllen. Dabei ist es teilweise erforderlich, dass diese Behältnisse vor deren Befüllung, insbesondere an deren Innenseiten, sterilisiert werden. Eine im Stand der Technik verbreite Methode ist hierbei die Sterilisation mittels sterilisierenden Gasen und insbesondere mittels Wasserstoffperoxid. In jüngerer Zeit ist man jedoch bemüht, den Einsatz von Chemikalien bei der Sterilisation von Behältnissen zu reduzieren. Daher sind in jüngerer Zeit auch Vorrichtungen und Verfahren bekannt geworden, welche die Behältnisse mit anderen Maßnahmen sterilisieren, wie beispielsweise mittels ultravioletter Strahlung oder Elektronenstrahlen.

In der noch unveröffentlichten europäischen Patentanmeldung Nr. 07 007 977.7 der Anmelderin sind ein Verfahren und eine Vorrichtung beschrieben worden, bei denen Strahlfinger, welche die Elektronenstrahlen abgeben, in ein Behältnis eingeführt werden und dabei zum Zwecke der Sterilisation das Behältnis relativ zu diesem Strahlfinger in der Längsrichtung der Behältnisse verschoben wird. Mit dieser Vorgehensweise ist eine effiziente Innenraumsterilisation von Kunststoffbehältnissen möglich. Gleichwohl ist jedoch zu beachten, dass zum Zwecke der Sterilisation die Behältnisse nicht in beliebiger Weise mit Strahlung beaufschlagt werden dürfen, sondern diese Strahlung hinsichtlich ihrer Intensität sowohl maximalen als auch minimalen Grenzwerten unterworfen ist. Die minimalen Werte werden dabei bestimmt durch diejenige Dosis, die nötig ist, um eine effiziente Sterilisation der Behältnisse zu erreichen, was wiederum von vielen Faktoren, beispielsweise von den abzutötenden Keimen, abhängt. Die maximale Dosis ist dadurch begrenzt, dass keine Beschädigungen an dem zu sterilisierenden Behältnis auftreten sollten.

Das Dokument WO 97/07024 zeigt das Sterilisieren des Inneren eines Behälters mittels eines Strahlfingers, der gemäß eines speziellen Zyklus bewegt wird.

Der vorliegenden Erfindung liegt daher die Aufgabe zugrunde, ein Verfahren und eine Vorrichtung zur Verfügung zu stellen, welche eine genauere Dosierung der aufgebrachten Strahlungsleistungen auf Behältnisse erlaubt. Damit soll weiterhin die Sterillisation von Behältnissen mittels Strahlung verbessert werden.

Dies wird erfindungsgemäß durch ein Verfahren nach Anspruch 1 und einer Vorrichtung nach Anspruch 9 erreicht. Vorteilhafte Ausführungsformen und Weiterbildungen sind Gegenstand der Unteransprüche.

Bei einem erfindungsgemäßen Verfahren zum Sterilisieren von Behältnissen wird ein Behandlungskopf in das Innere des zu sterilisierenden Behältnisses geführt und der Behandlungskopf emittiert im Inneren des Behältnisses während eines vorgegebenen Zeitraums Strahlung, wobei das Behältnis gegenüber dem Behandlungskopf in einer Längsrichtung des Behältnisses während des vorgegebenen Zeitraums wenigstens zeitweise mit einer relativen Bewegungsgeschwindigkeit bewegt wird. Erfindungsgemäß variiert die relative Bewegungsgeschwindigkeit des im Inneren des Behältnisses befindlichen Behandlungskopfes während des vorgegebenen Zeitraums und wird in Abhängigkeit eines Innenprofils des Behältnisses mittels des gemäß Anspruch 1 ermittelten Fahrprofils gesteuert.

Damit wird erfindungsgemäß vorgeschlagen, die Bewegungsgeschwindigkeit des Behandlungskopfes gegenüber dem Behältnis und damit auch die Bewegungsgeschwindigkeit derjenigen Stelle, an der die besagte Strahlung austritt, in Abhängigkeit von einem Profil des Behältnisses zu steuern. Unter einer Variation der Bewegungsgeschwindigkeit wird dabei eine Variation der Bewegungsgeschwindigkeit in einer bestimmten Bewegungsrichtung verstanden und insbesondere nicht ein Vorzeichenwechsel der Bewegungsgeschwindigkeit.

Dabei ist es sowohl möglich, dass der Behandlungskopf ruht und sich demgegenüber das Behältnis in seiner Längsrichtung bewegt. Auch wäre es möglich, dass der Behandlungskopf in einer Längsrichtung des Behältnisses bewegt wird oder auch, dass sich sowohl das Behältnis als auch der Behandlungskopf bewegen.

Bevorzugt finden dabei zumindest zeitweise und bevorzugt vollständig die Emission von Strahlung und die Bewegung in der Längsrichtung gleichzeitig statt. Es wäre jedoch auch möglich, dass die Bewegung stückweise erfolgt und jeweils in den Ruhepausen Strahlung emittiert wird.

Durch die Änderung der Bewegungsgeschwindigkeit kann eine Anpassung an unterschiedliche Profile der Flasche erreicht werden. So ist es beispielsweise möglich, dass der Behandlungskopf, während er die Mündung des Behältnisses passiert, schneller bewegt wird, da in diesem Falle die Innenwandung des Behältnisses nahe an dem Behandlungskopf liegt. Unter dem Innenprofil des Behältnisses wird dabei insbesondere die Innenwandung des Behältnisses, insbesondere in Bezug auf den Behandlungskopf verstanden.

Allgemein wird während der Bewegung wenigstens ein Parameter gesteuert, der für eine auf die Innenfläche bezogene Strahlungsleistung charakteristisch ist. So wäre es auch möglich, anstelle der Bewegungsgeschwindigkeit die von dem Behandlungskopf abgegebene Leistung zu steuern, beispielsweise über eine Änderung der Beschleunigungsspannung im Falle von Elektronenstrahlern. Daneben könnte auch eine Gegenspannung verwendet werden.

Weiterhin wäre es auch möglich, in Abhängigkeit von dem Innenprofil des Behältnisses eine höhere oder geringere Absorption der Strahlung zu erreichen, bevor diese auf das Behältnis gelangt. Dies wäre beispielsweise dadurch möglich, dass eine Kühlung des Behandlungskopfes bewusst zu bestimmten Zeiten vorgenommen wird und das Kühlmedium die austretende Strahlung bzw. die auf die Innenwandung des Behältnisses auftreffende Strahlung abmindert. Auch könnten Absorptionselemente vorgesehen sein, welche die Strahlung in vorbestimmter und veränderbarer Weise absorbieren.

Bei einem bevorzugten Verfahren emittiert der Behandlungskopf Ladungsträger. Dabei kann der Behandlungskopf oder auch ein anderes Element eine Elektronenemissionsquelle aufweisen, sowie eine Beschleunigungseinrichtung, welche diese Elektronen hin zu einem Austrittsfenster beschleunigt. Mit Hilfe dieser austretenden Elektronen wird die Sterilisation der Behältnisinnenwandungen bewirkt.

Bei einem weiteren bevorzugten Verfahren wird die Bewegung durch eine Steuereinrichtung auf Grundlage von in einer Speichereinrichtung abgelegten Daten gesteuert, wobei diese Daten für Querschnitte des Behältnisses in der Längsrichtung des Behältnisses charakteristisch sind. So kann beispielsweise in denjenigen Abschnitten des Behältnisses, in denen dieses einen größeren Querschnitt aufweist, die Bewegungsgeschwindigkeit des Behandlungskopfes gegenüber dem Behältnis reduziert werden und in denjenigen Abschnitten, in denen der Querschnitt verringert ist, die Bewegungsgeschwindigkeit des Behandlungskopfes gegenüber dem Behältnis erhöht werden.

So ist es möglich, dass diese Relativbewegung zwischen dem Behandlungskopf und dem Behältnis über vorgegebene Fahrprofile programmiert wird. Dabei ist es möglich, für den gesamten Bestrahlungsprozess ein gesamtes Fahrprofil zu erstellen und dieses bevorzugt in einzelne Kurvensegmente zu zerlegen. Dabei können den einzelnen Hubbewegungen des Behältnisses unterschiedliche Hubgeschwindigkeiten und gegebenenfalls auch Beschleunigungen zugewiesen werden. Diese Aufteilung ist vorteilhaft, um eine bessere Anpassung an die Geometrie des Behältnisses zu erhalten.

Dabei ist es möglich, für ein derartiges Kurvensegment Start-und Endpunkt d.h. den Hubweg anzugeben sowie auch die erforderliche Fahrgeschwindigkeit d.h. die Hubgeschwindigkeit. Diese einzelnen Kurvensegmente lassen sich anschließend zu einer gesamten Kurve, welche dem Fahrprofil entspricht, zusammenfügen. Dabei können die Hubwege und die Fahrdauer der einzelnen Kurvensegmente aufaddiert werden, so dass sie in der Gesamtheit den Gesamthub und die Gesamtprozessdauer des Fahrprofils ergeben. Dabei ist es möglich, dass sich zwischen den einzelnen Segmenten Knickpunkte ergeben, in denen unterschiedliche Geschwindigkeiten der Fahrprofile ineinander übergehen. Es wäre jedoch auch möglich, über eine weitere Steuerung derartige Knickpunkte zu vermeiden und durch entsprechende Beschleunigungen und Verzögerungen ein glattes (d.h. mathematisch gesehen differenzierbares) Geschwindigkeitsprofil zu erreichen.

Bei einem weiteren bevorzugten Verfahren wird die Bewegung des Behandlungskopfes in Abhängigkeit von einem Durchmesser des Behältnisses gesteuert. Hierbei ist zu beachten, dass insbesondere aus dem Behandlungskopf austretende Elektronen eine bestimmte Reichweite haben bzw. einen bestimmten Anteil ihrer Energie über die zurückgelegte Weglänge verlieren. Insbesondere in Bereichen großer Durchmesser des Behältnisses ist es daher erforderlich, dass die Einwirkzeit entsprechend länger ist, um die entsprechend geringere Energie der Elektronen zu kompensieren.

Bei einem weiteren bevorzugten Verfahren wird die Bewegung des Behandlungskopfes auch in Abhängigkeit von einem Winkel gesteuert, unter dem sich die Innenwandung des Behältnisses gegenüber der Längsrichtung des Behältnisses erstreckt. So ist beispielsweise im Mündungsbereich eines Behältnisses oft ein sich von oben nach unten erweiternder Behältnisabschnitt vorgesehen, der von der austretenden Elektronenstrahlung nur schwer erreichbar ist. Auch in diesen Bereichen sollte demnach die Bewegungsgeschwindigkeit reduziert werden.

Wie oben erwähnt, wird besonders bevorzugt die Bewegung aufgrund eines programmierten Fahrprofils gesteuert. Dieses Fahrprofil kann dabei aufgrund empirischer Daten für entsprechende Behältnisse ermittelt werden, es wäre jedoch auch möglich, dieses Fahrprofil aufgrund von Parametern des Behältnisses, wie beispielsweise der Krümmung und dem Querschnitt, zu ermitteln.

Bevorzugt wird das Behältnis selbst in seiner Längsrichtung bewegt und damit besonders bevorzugt der Behandlungskopf bzw. der Strahlfinger höhenkonstant gehalten. Zu diesem Zweck kann eine Trägervorrichtung vorgesehen sein, an der wiederum eine Klammer angeordnet ist, welche das Behältnis selbst und in seiner Längsrichtung nach oben oder unten bewegt.

Bei einem bevorzugten Verfahren wird an der Innenwandung des Behältnisses ein Material aufgebracht, welches auf die in den Behandlungskopf ausgesandte Strahlung reagiert und auf diese Weise Rückschlüsse auf die Intensität der Strahlung zulässt.

So ist es beispielsweise möglich, eine Dosismessung mit Hilfe einer bestrahlten Messfolie durchzuführen, wobei diese Messfolie an der Innenwandung des Behältnisses angebracht wird. Auf diese Weise kann die Dosisverteilung in den behandelten Behältnissen ermittelt werden. Diese Messstreifen verfärben sich dabei abhängig von der Dosisleistung und können mit einem Dosismessgerät ausgewertet werden. Dabei ist es möglich, dass die Messfolie durch einen entsprechenden Kunststoffträger kaschiert ist, um auf diese Weise auch die Effekte zu simulieren, welche im Inneren der Behälterwandung auftreten. Zusätzlich kann auf der Messfolie ein Farbstoff appliziert werden. Unter Einwirkung der Strahlung verfärbt sich die Folie proportional zu der absorbierten Energiemenge in bestimmten Farbtönen. Diese Farbtöne können anschließend mit einem Dosismessgerät ausgelesen werden und geben damit an, welche Dosis beispielsweise in Kilogray (kGy) aufgebracht wurde.

Bei einem weiteren erfindungsgemäßen Verfahren wird ein Fahrprofil für die Innensterilisation von Behältnissen bestimmt, wobei ein Behandlungskopf in das Innere des Behältnisses geführt wird und der Behandlungskopf im Inneren des Behältnisses Strahlung auf eine Innenwandung des Behältnisses richtet, wobei das Behältnis gegenüber dem Behandlungskopf, bevorzugt während der Emission der Strahlung, in einer Längsrichtung des Behältnisses bewegt wird. Erfindungsgemäß wird eine Vielzahl von Daten aufgenommen, welche für eine auf eine Innenwandung des Behältnisses auftreffende Strahlung charakteristisch sind. Dieses Bestimmungsverfahren ist dabei eng mit der oben genannten Erfindung korreliert, da insbesondere mit den so ermittelten Daten das oben erwähnte Fahrprofil für den späteren Arbeitsbetrieb ermittelt werden kann.

Es ist jedoch auch möglich, dieses Messverfahren unabhängig von dem eigentlichen, oben beschriebenen, Arbeitsverfahren, auszuführen. Bevorzugt wird eine Vielzahl von Behältnissen mit dem beschriebenen Messverfahren vermessen und die Daten dieser Behältnisse werden in einer Datenbank abgelegt, welche bei dem späteren Arbeitsverfahren verwendet werden kann. Dabei ist es auch möglich, neue Behältnisse, welche bereits vermessenen Behältnissen ähneln, durch Anpassung der für dieses vermessene Behältnis ermittelten Werte zu behandeln. Auch wäre es möglich, die zu vermessenden Behältnisse in Abschnitte einzuteilen, etwa nach ähnlichen Kopf- oder Bodenabschnitten und auf diese Weise eine Anwendung der gewonnenen Messwerte auf eine größere Vielzahl von Behältnissen zu ermöglichen, etwa, wenn ein bestimmtes Behältnis hinsichtlich seines Kopfbereiches einem ersten vermessenen Behältnis ähnelt und hinsichtlich seines Bodenbereichs einem zweiten Behältnis.

Die vorliegende Erfindung ist weiterhin auf eine Vorrichtung zum Sterilisieren von Behältnissen gerichtet, wobei diese Vorrichtung einen Behandlungskopf aufweist, der derart gestaltet ist, dass er in einem Innenraum eines zu sterilisierenden Behältnisses führbar ist. Dabei weist dieser Behandlungskopf eine Strahlungsquelle auf, welche Strahlung ausgibt, und die Vorrichtung weist eine Bewegungseinrichtung auf, welche das Behältnis gegenüber dem Behandlungskopf in einer Längsrichtung des Behältnisses mit einer vorgegebenen Bewegungsgeschwindigkeit bewegt, während der Behandlungskopf wenigstens zeitweise Strahlung abgibt. Erfindungsgemäß weist die Vorrichtung eine Steuereinrichtung auf, welche diese relative Bewegungsgeschwindigkeit des im Inneren des Behältnisses befindlichen Behandlungskopfes variiert und in Abhängigkeit eines Innenprofils des Behältnisses steuert.

Bevorzugt handelt es sich um einen Behandlungskopf, der durch eine Mündung in das Behältnis einführbar ist, wobei diese Mündung einen geringeren Querschnitt aufweist, als beispielsweise ein Grundkörper des Behältnisses. Bei der Bewegungseinrichtung kann es sich dabei um einen Antrieb, wie insbesondere aber nicht ausschließlich um einen Linearmotor handeln, der das Behältnis in Abhängigkeit von einem vorbestimmten Fahrprofil bewegt.

Vorzugsweise sendet die Strahlungsquelle Ladungsträger und insbesondere Elektronen aus. Es wäre jedoch auch möglich, dass eine andere Art von Strahlung, wie beispielsweise Röntgenstrahlung, UV-Strahlung oder dergleichen ausgesandt wird. Das Grundkonzept dieser Erfindung lässt sich auch auf diese anderen Strahlungsarten anwenden.

Bei einer weiteren vorteilhaften Ausführungsform weist die Vorrichtung eine Speichereinrichtung auf, in der ein für die Bewegung des Behandlungskopfes charakteristisches Fahrprofil gespeichert ist. Dieses Fahrprofil kann sich dabei, wie oben erwähnt, aus empirischen Daten ergeben, die für einen bestimmten Flaschentyp bestimmt wurden. Es wäre jedoch auch möglich, dass sich das Fahrprofil teilweise aufgrund von theoretischen Überlegungen ergibt, welche beispielsweise den Querschnitt eines Behältnisses und auch den Neigungswinkel der Behältniswandung berücksichtigen.

Erfindungsgemäß weist die Vorrichtung eine Bildaufnahmeeinrichtung auf, welche ein Bild eines zu sterilisierenden Behältnisses aufnimmt. Dabei in nicht erfindungsgemässer Weise die Daten dieser Bildaufnahme für die Geschwindigkeitssteuerung verwendet . So ist es beispielsweise möglich, dass in einem Arbeitsbetrieb ein Bild eines Behältnisses aufgenommen wird und anschließend eine Bildverarbeitungssoftware dieses Bild mit einer Vielzahl von Bildern für abgespeicherte Behältnisse vergleicht. Sobald ein entsprechendes Behältnis gefunden wurde kann ein entsprechendes Fahrprofil geladen werden und im Arbeitsbetrieb aufgrund dieses Fahrprofils die Bewegung des Behandlungskopfes gesteuert werden.

Es wäre jedoch auch in nicht erfindungsgemässer Weise möglich, dass ein Bild aufgenommen wird und anhand dieses Bildes die Geometrien des Behältnisses ermittelt und unter Zugrundelegung dieser Geometrien direkt das Fahrprofil für den Behandlungskopf gesteuert wird. Auch ist es möglich, dass sowohl das Fahrprofil aufgrund von direkten geometrischen Überlegungen gesteuert wird als auch aufgrund von gemäß Anspruch 8 ermittelten empirischen Daten.

Bei einem weiteren bevorzugten Verfahren weist die Vorrichtung eine Vielzahl von Behandlungsköpfen auf, welche hintereinander an einer Transporteinrichtung angeordnet sind. Dabei ist es möglich, dass sowohl Flaschenhalterungen wie beispielsweise Greifelemente, als auch die Behandlungsköpfe an der gleichen Transporteinrichtung angeordnet sind und damit synchron zueinander bewegt werden. Vorzugsweise werden die Behältnisse während ihrer Sterilisation bewegt, beispielsweise auf einem Transportkarussell, an dem auch die entsprechenden Behandlungsköpfe mitbewegt werden. Auf diese Weise kann ein hoher Durchsatz an Behältnissen hintereinander sterilisiert werden.

Dabei ist es sowohl möglich, bereits fertig geformte Behältnisse innen zu sterilisieren, als auch Vorformlinge vor deren Umformung zu Behältnissen.

Damit kann eine derartige Vorrichtung sowohl als Rundläufer als auch als Lineareinheit ausgeführt sein. Dabei ist es möglich, dass die gesamte Vorrichtung in einem Behandlungsraum untergebracht ist, der sowohl der Innen- als auch der Außensterilisation von Behältnissen und insbesondere von PET-Behältnissen durch Elektronenstrahlung dient. Die Sterilisationseinheit selbst kann dabei auch in eine Streckblasmaschine oder in ein Füllaggregat integriert sein oder auch als einzeln stehende Einheit angeordnet sein. Letztere Variante bietet den Vorteil, dass bestehende Linien eventuell nachgerüstet werden können. Die Integration in eine Streckblasmaschine oder in eine Fülleinrichtung bietet den Vorteil, dass auf diese Weise die Kosten für die Gesamtherstellung reduziert werden können.

Auch ist es möglich, dass die Behältnisse über Sterne, beispielsweise Sterne im Neck-handling an die Sterilisationseinheit übergeben werden, wo sie mittels einer linearen Hubbewegung über den Behandlungskopf bzw. Strahlfinger bewegt werden.

Vorzugsweise dient als Strahlerzeuger eine kompakte Elektronenstrahleinheit mit einem Strahlfinger, der wie oben erwähnt, derart dimensioniert ist, dass dieser in die Flasche eintauchen kann, um die Elektronenwolke mit möglichst geringer Energie auf die innere Oberfläche der Behältnisse anzuwenden. Im Rahmen einer Rundläuferanordnung wäre es dabei möglich, dass entsprechende Transformatoren bzw. Netzgeräte für die Elektronenstrahlerzeuger auf einem Karussell mitlaufen, wodurch die Zuführung der Hochspannung vereinfacht wird.

Die Möglichkeit einer verbesserten Oberflächenbehandlung bzw. Sterilisation für insbesondere auch komplex geformte Getränkebehältnisse durch Elektronenstrahlen kann durch die Verknüpfung elektronischer und mechanischer Komponenten realisiert werden. Auf diese Weise kann trotz der dreidimensionalen Ausdehnung der Behälntisse eine möglichst homogene Dosisverteilung auf der Innenoberfläche der Behältnisse erreicht werden. Das zu behandelnde Substrat bzw. der Behälter wird bevorzugt relativ zu einer feststehenden Strahlungsquelle bewegt, wobei, wie oben erwähnt, der Bewegungsablauf an die aufgebrachte Dosisleistung angepasst wird.

Weitere Vorteile und Ausführungsformen ergeben sich aus den beigefügten Zeichnungen:

Darin zeigen:
- Fig. 1: Eine schematische Darstellung einer erfindungsgemäßen Sterilisationseinrichtung;
- Fig. 2: ein Beispiel für ein Fahrprofil eines Behandlungskopfes;
- Fig. 3a: ein Beispiel für eine Strahlungsverteilung im Inneren eines Hohlbehältnisses;
- Fig. 3b: ein weiteres Beispiel für eine Strahlungsverteilung im Inneren eines Hohlbehälters;
- Fig. 4: ein Beispiel für eine Strahlungsbehandlung im Inneren eines zylinderförmig ausgebildeten Behältnisses;
- Fig. 5: eine schematische Darstellung zur Veranschaulichung der relevanten Strahlungsparameter;
- Fig. 6: eine schematische Darstellung einer Vielzahl von auf einer Transporteinrichtung angeordneten erfindungsgemäßen Vorrichtungen; und
- Fig. 7: eine Darstellung einer Anlage mit einer Vielzahl von erfindungsgemäßen Vorrichtungen.

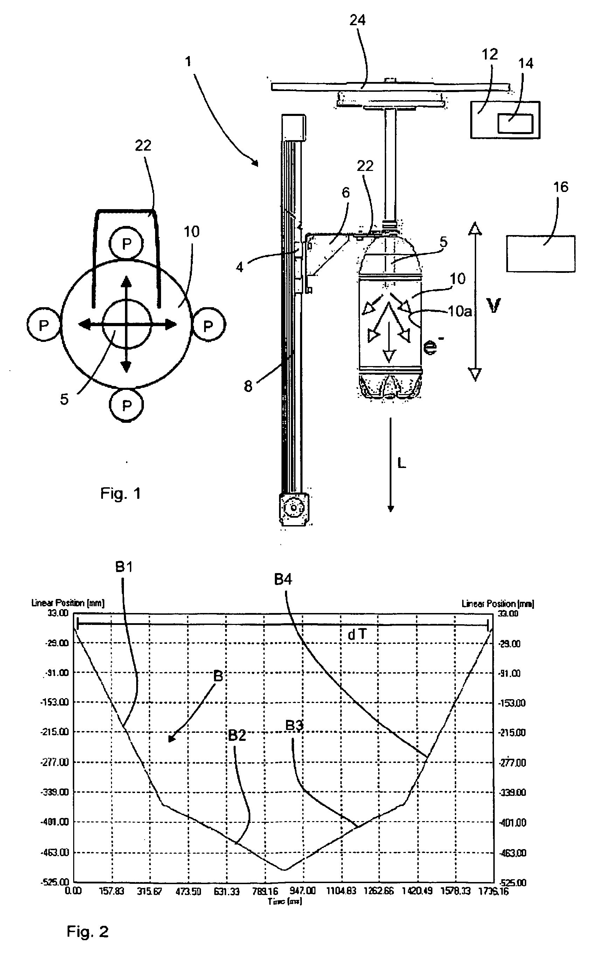

Fig. 1 zeigt eine schematische Darstellung einer erfindungsgemäßen Vorrichtung 1, welche Bestandteil einer Anlage zum Behandeln von Behältnissen sein kann. Dabei bezieht sich das Bezugszeichen 10 auf ein Behältnis, welches eine Innenwandung 10a aufweist, welche mit Hilfe von durch e-gekennzeichneten Elektronen zu sterilisieren ist. Das Bezugszeichen 5 kennzeichnet einen Behandlungskopf, der in das Innere des Behältnisses 10 einführbar ist. Dabei ist hier dieser Behandlungskopf 5 in der Längsrichtung L des Behältnisses stationär angeordnet bzw. an einem Träger 24 angeordnet. Die Elektronen werden unter Hochvakuum beschleunigt und über ein (hier nicht im Detail gezeigtes) Elektronenaustrittsfenster ausgesandt. Die Elektronen werden an Luftmolekülen gestreut, so dass sich eine Elektronenwolke ausbildet, welche zur Sterilisation der Innenwandung 10a des Behältnisses 10 dient.

Bei einer weiteren bevorzugten Ausführungsform weist die Vorrichtung bzw. der Behandlungskopf 5 ein Innengehäuse auf, innerhalb dessen die Beschleunigungseinrichtung angeordnet ist und ein Außengehäuse umgibt dieses Innengehäuse. Der Innenraum dieses Innengehäuses, in dem bevorzugt die Ladungsträgererzeugungsquelle und die Beschleunigungseinrichtung vorgesehen sind, werden besonders bevorzugt mit einem Vakuum beaufschlagt. Besonders bevorzugt wird zwischen dem Außengehäuse und dem Innengehäuse ein sich bis zu dem Behandlungskopf erstreckender Raum gebildet, durch den ein Medium und insbesondere ein gasförmiges oder flüssiges Medium führbar ist.

Dieses gasförmige Medium dient insbesondere zum Kühlen des Austrittfensters und zu diesem Zweck wird das gasförmige oder flüssige Medium entlang des Innengehäuses bis zu dem Behandlungskopf und auch vorbei an dem Austrittsfenster geführt. Vorzugsweise wird damit zwischen dem Außengehäuse und dem Innengehäuse ein Raum in Form eines Kanals oder mehrerer Kanäle gebildet, wobei besonders bevorzugt ein unterer Endabschnitt des äußeren Gehäuses so gestaltet ist, dass der Gasstrom auch beispielsweise in radialer Richtung der Vorrichtung und damit auch an dem Austrittsfenster vorbeigeführt wird. Es wird darauf hingewiesen, dass der Strom des gasförmigen Mediums nicht und zumindest nicht unmittelbar an die Innenwandung des Behältnisses gelangt, sondern, wie gesagt, insbesondere dazu dient, um an dem Austrittsfenster vorbeigeführt zu werden.

Das Behältnis 10 wird von einer Greifklammer 22 gehalten, die an einem Träger 6 angeordnet ist, wobei dieser wiederum an einem Linearantrieb 4 angeordnet ist. Dabei bezieht sich das Bezugszeichen 8 auf einen Halter, entlang dessen der Linearantrieb 4 in der Längsrichtung L bewegbar ist. Damit wird auch das Behältnis 10 in der Längsrichtung L bewegt. Bevorzugt handelt es sich, wie oben erwähnt, bei dem Antrieb um einen Linearmotor. Dabei wäre es möglich, diesen Linearmotor auch unterhalb des Behältnisses anzuordnen oder auch oberhalb des Behandlungskopfes, so dass keine Ablenkung bzw. einseitige Ablenkung der Elektronen durch den Linearmotor auftritt. Die Klammer 22 und auch der Träger 6 können dabei aus einem Kunststoff ausgeführt sein, der keine Kräfte auf Elektronen ausübt. Das Bezugszeichen v bezieht sich auf die Geschwindigkeit, in der die Relativbewegung zwischen dem Behandlungskopf 5 und dem Behandlungsbehältnis 10 auftritt.

Damit tritt eine Relativbewegung der Flasche in dem Elektronenstrahl auf, wobei hierdurch eine Anpassung der Dosisverteilung durch ein programmierbares Fahrprofil möglich ist. Die in Fig. 1 gezeigte Vorrichtung kann auch zur Bildung eines Referenzprofils verwendet werden. zu diesem Zweck können auf der Innenoberfläche der Behältnisse 10 ein oder mehrere Messstreifen angebracht werden. Dabei zeigt bevorzugt der Messstreifen in die Bestrahlungsrichtung, die ausgewertet werden soll. Dabei ist es beispielsweise möglich, die Innenwandung des Behältnisses in vier Strahlungspositionen P einzuteilen, welche in dem linken Teilbild von Fig. 1 dargestellt sind.

An der oberen Position ist dabei das Greifelement für das Behältnis angeordnet und die weiteren Positionen werden im Uhrzeigersinn um die Innenwanndung des Behältnisses verteilt. Wie oben erwähnt, besteht das Ziel für diese Optimierung der Fahrprofile darin, dass weder zu viel Dosis noch zu wenig Dosis in den ausgewählten Flaschen appliziert wird. Dabei soll weiterhin die Behandlungszeit der Behältnisse und die Strahlleistung so gering wie möglich gehalten werden.

Vorteilhaft ist die gesamte Bewegung in der Längsrichtung des Behältnisses innerhalb von ein bis zwei Sekunden beendet. Die Behandlungszeit bezieht sich dabei auf den Zeitraum, in dem sich der Behandlungskopf in der Flasche befindet. Damit setzt sich ein gesamter Zyklus aus einer Anfahrts-, einer Behandlungs- und einer Abfahrtszeit zusammen. Es ist dabei möglich, dass die Strahlung sowohl während der Bewegung ausgegeben wird als auch die Bewegung sukzessive angehalten wird und während der Ruhephasen Strahlung ausgegeben wird.

Weiterhin ist bevorzugt eine (nicht gezeigte) Abschirmung, insb. aus Blei, vorgesehen, um entstehende Röntgenstrahlung abzuschirmen. Daneben können entstehende Gase wie Ozon und Stickoxide abgesaugt werden.

Um einen Anhaltspunkt für die Dosisverteilung in dem Behältnis zu erreichen, wird das Behältnis zunächst in einer konstanten Geschwindigkeit über den Behandlungskopf gefahren. Anschießend wird das Behältnis ohne Haltepause am Endpunkt unter gleichen Strahlungsbedingungen in ihre Grundposition zurückverfahren. Durch diese Vorgehensweise kann mit Hilfe der Dosiermeterfolie ein Strahlungsprofil für das Behältnis unter den vorgegebenen Bedingungen ermittelt werden. Dieses Strahlungsprofil kann anschließend abgespeichert und bei der Behandlung künftiger Behältnisse zugrundegelegt werden. Das Bezugszeichen 16 bezieht sich auf eine Bildaufnahmeeinrichtung zum Aufnehmen von Bildern der Behältnisse. Auf Grundlage solcher Bilder können die Behältnisse identifiziert werden und entsprechend eines identifizierten Behältnisses die korrekten Fahrprofile geladen werden.

Das Bezugszeichen 12 bezieht sich auf eine (nur schematisch gezeigte) Steuereinrichtung, welche die Bewegung des Behältnisses in der Längsrichtung steuert. Dabei ist ebenfalls eine Speichereinrichtung 14 vorgesehen, in welcher Fahrprofile zum Steuern der Bewegung abgelegt sind. Diese Steuerungseinrichtung kann auch die Steuerung der Emission von Elektronen durch den Behandlungskopf 5 übernehmen.

Fig. 2 zeigt ein Bewegungsprofil für ein Behältnis gegenüber einem Strahlfinger. Dabei ist auf der linken Koordinate eine Linearposition in Millimetern aufgetragen und auf der Ordinate ist eine Zeit in Millisekunden aufgetragen. In diesem Falle setzt sich das gesamte Fahr- bzw. Bewegungsprofil B aus vier Segmenten B1 - B4 zusammen, wobei hier ein symmetrisches Bewegungsprofil verwendet wird. Die einzelnen Segmente B1 - B4 gehen über Knickstellen ineinander über, wobei dies jedoch, wie oben erwähnt, nicht zwingend erforderlich ist.

Durch eine entsprechende Geschwindigkeitssteuerung kann vielmehr auch eine ruckfreie Bewegung erreicht werden. Bei der in Fig. 2 gezeigten Darstellung kehrt sich die Bewegungsrichtung zwischen den Segmenten B2 und B3 unmittelbar um. Es wäre jedoch auch möglich, dass das Behältnis für kurze Zeit stillsteht und während dieser Zeit Elektronenstrahlung emittiert wird. Das Bezugszeichen dT kennzeichnet den Zeitraum, innerhalb dessen die Sterilisation der Behältnisse 10 stattfindet. Innerhalb dieses Zeitraums findet damit sowohl die Relativbewegung des Behandlungskopfes 5 gegenüber dem Behältnis 10 als auch die Emission von Elektronen statt

Dabei ist es möglich, dass das gesamte Fahrprofil B unter Zugrundelegung von Messungen ermittelt wird, welche an einem bestimmten Behältnis durchgeführt wurden. Es wäre jedoch auch denkbar, dass unterschiedliche Segmente auf an unterschiedlichen Behältnissen durchgeführten Messungen basieren. So beschreiben beispielsweise die Segmente B1 und B4 die Bewegung des Behandlungskopfes 5 in einem oberen Bereich des Behältnisses 10 und die Segmente B2 und B3 die Bewegung in einem unteren Bereich des Behältnisses. Es wäre hier möglich, dass die Segmente B1 und B4 auf Messungen an einem ersten Referenzbehältnis und die Segmente B2 und B3 auf Messungen an einem zweiten Referenzbehältnis basieren.

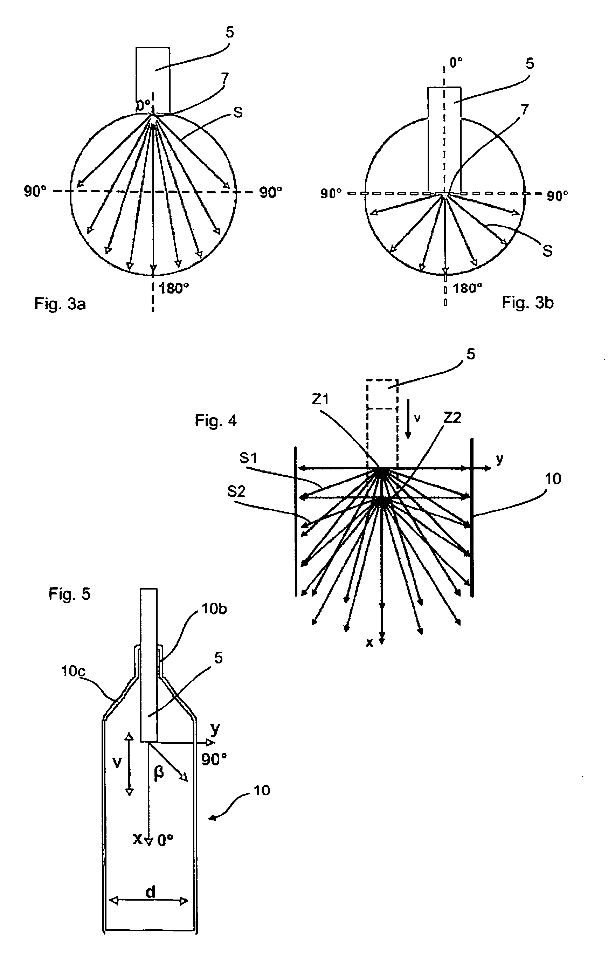

Die Figuren 3a und 3b zeigen einen Messaufbau für ein Behältnis mit kugelförmigem Innenquerschnitt. Dabei ist in der linken Teilfigur der Behandlungskopf 5 bzw. dessen Austrittsfenster 7 an dem oberen Ende des Behältnisses 10 angebracht und in Fig. 3b im Zentrum des Behältnisses 10. Man erkennt, dass sich auch in Abhängigkeit von der Position des Behandlungskopfes 5 gegenüber dem Innenraum des Behältnisses unterschiedliche Strahlverteilungen S ergeben. Insbesondere sind die Weglängen der Strahlung bei der in Fig. 3b gezeigten Variante kürzer als bei der in Fig. 3a gezeigten Position und damit tritt im Falle der in Fig. 3b gezeigten Ausführungsform eine intensivere Bestrahlung der unteren Halbkugel des Behältnisses 10 auf.

Nachdem die in den Figuren 3a und 3b gezeigten Experimente durchgeführt worden sind, können die im Inneren der Behältnisse angebrachten Dosimeterfolien entfernt und ausgewertet werden.

In den Figuren 4 und 5 ist ein weiterer Versuch zur Ermittlung eines Fahrprofils dargestellt. Es ergibt sich, dass die Dosisverteilung von den Prozessparametern Hubgeschwindigkeit (v), Flaschengeometrie (d, β), Beschleunigungsspannung, Strahlstrom und Behandlungsdauer abhängig ist. Dabei steht die Geschwindigkeit v in Korrelation mit dem Durchmesser d und dem Öffnungswinkel β des Behältnisses. Da in Abhängigkeit von der Geometrie des Behältnisses eine homogene Dosisverteilung bei konstanter Hubgeschwindigkeit nicht auftritt, wird, wie oben erwähnt, die Hubgeschwindigkeit variiert.

In Fig. 4 ist dabei ein Behandlungskopf 5 in zwei unterschiedlichen Positionen dargestellt, d.h. das jeweilige Zentrum Z1, Z2, in dem sich das Austrittsfenster befindet, ist in Fig. 4 an zwei unterschiedlichen Positionen dargestellt. Entsprechend ergeben sich auch unterschiedliche Strahlverteilungen S1 und S2 an der Innenwandung des Behältnisses. Ausgehend von der Startposition in Fig. 4 d.h. der oberen Position des Behandlungskopfes wird durch die Streubirne die Dosis auf der Flaschenwand appliziert. Wenn der Strahlfinger mit einer Geschwindigkeit v in die Zielposition fährt, bewegt sich auch diese Streubirne und die neu applizierte Dosis wird mit der Dosis aus der Startposition überlagert. Damit hängt die erreichbare Dosis auf der Flascheninnenwandung auch von der zuvor durch den Behandlungskopf applizierten Dosis ab. Das Bezugszeichen 10b in Fig. 5 bezieht sich auf eine Mündung des Behältnisses und das Bezugszeichen 10c auf den unter dieser Mündung 10b liegenden Mündungsbereich des Behältnisses 10 innerhalb dessen sich der Querschnitt d des Behältnisses 10 erweitert.

Die Höhe der Strahlleistung d.h. die Beschleunigungsspannung und der Strahlstrom richten sich nach der zu behandelnden Flasche und dem zu behandelnden Referenzkeim. Je größer die Resistenz dieses Keims und die Flaschendimensionen sind, desto höher muss die Strahlleistung eingestellt werden. Für die Stärke des Elektronenaustrittsfensters 7 erweist sich eine Titanfolie in einer Dicke in einem Bereich von 7-13 µm als besonders geeignet. Diese Stärke gewährleistet mittels aktiver Luftkühlung eine ausreichende Stabilität gegenüber dem Wärmeeintrag des Elektronenstrahls.

Des Weiteren wirkt sich die Titanfolie mit zunehmender Dicke negativ auf das Sterilisationsergebnis aus. Eine dickere Folie absorbiert mehr Strahlungsenergie als eine dünnere und folglich steht in diesem Fall weniger Energie zur Dosisapplizierung zur Verfügung.

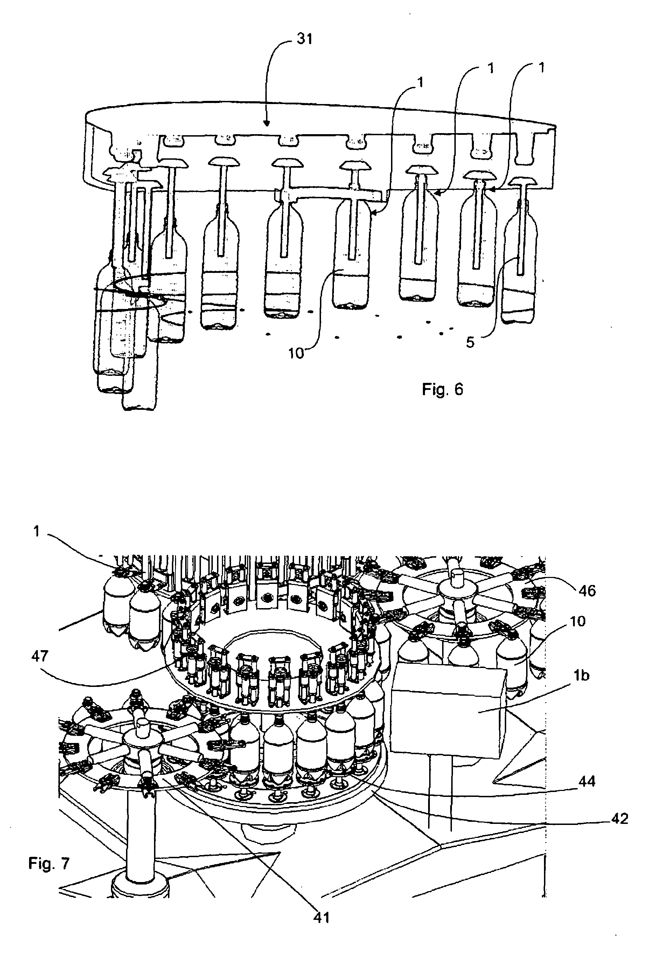

Fig. 6 zeigt eine schematische Darstellung des Umlaufrades 31. Dieses Umlaufrad 31 weist eine Vielzahl von erfindungsgemäßen Vorrichtungen 1 auf, die jeweils auf der gleichen Höhe angeordnet sind, man erkennt jedoch, dass die einzelnen Behältnisse 10 jeweils bis zu einer Maximalposition angehoben werden und auf diese Weise der Behandlungskopf bzw. der Strahlfinger 5 in Abhängigkeit von der Drehstellung unterschiedlich tief in die Behältnisse 10 eingetaucht wird. Während dieses gesamten Vorgangs sind die erfindungsgemäßen Vorrichtungen 1 aktiviert und auf diese Weise wird die Innenwandung der Behältnisse 10a über die gesamte Höhe der Behältnisse 10 sterilisiert. Die Länge der Strahlfinger 5 wird dabei so gewählt, dass auch eine effektive Sterilisation der Böden der Behältnisse 10 möglich ist.

Fig. 7 zeigt eine perspektivische Ansicht einer erfindungsgemäßen Anordnung. Stromaufwärts bezüglich einer Vielzahl von erfindungsgemäßen Vorrichtungen 1 ist ein weiteres Transportkarussell 42 vorgesehen, auf dem die Behältnisse transportiert werden. Dabei werden die Behältnisse an einer weiteren Sterilisationsvorrichtung 1b für Behältnisse vorbeigeführt und von dieser weiteren Sterilisationsvorrichtung 1b an ihrer Außenwandung sterilisiert. Anschließend wird, wie oben beschrieben, der Innenraum der Behältnisse 10 sterilisiert.

Das Bezugszeichen 44 bezieht sich auf Dreheinrichtungen wie Drehteller, mittels denen die Behältnisse 10 um ihre Längsrichtung (bzw. Längsachse) gedreht werden. Durch diese Drehung kann ein größerer Bereich des Außenumfangs der Behältnisse 10 sterilisiert werden. Auch wäre es möglich, in der Transportrichtung der Behältnisse 10 mehrere Sterilisationsvorrichtungen 1b hintereinander vorzusehen.

Der Elektronenstrahlvorrichtung wurde detailliert in der europäischen Patentanmeldung Nr. 07 007 977.7 der Anmelderin beschrieben. Der Inhalt dieser Patentanmeldung wird hiermit durch Bezugnahme auch vollständig zum Offenbarungsgehalt der vorliegenden Anmeldung gemacht.

Es wäre weiterhin auch möglich, dass das Behältnis 10 während der Emission der Elektronenstrahlung unter ein (Teil)vakuum gesetzt wird. Durch diese Vorgehensweise kann die Reichweite der Elektronen nach dem Austritt aus dem Austrittsfenster 7 erhöht werden. Dabei ist es auch möglich, dass ein derartiges Beaufschlagen mit einem Teilvakuum bereits vor dem Beginn der Elektronenemission stattfindet. Diese Vorgehensweise kommt sowohl bei der Sterilisation von Vorformlingen als auch bei der Sterilisation von bereits geformten Behältnissen 10 in Betracht.

Um ein derartiges (Teil-)Vakuum im Inneren der Behältnisse 10 zu erreichen, könnte beispielsweise an dem Außenumfang des Behandlungskopfes 5 eine Dichtungseinrichtung (nicht gezeigt) angeordnet sein, die einerseits gegenüber dem Behandlungskopf 5 in dessen Längsrichtung beweglich ist und andererseits bei einem Eintreten des Behandlungskopfes 5 in das Behältnis 10 den Raum zwischen dem Behandlungskopf 5 und der Mündung 10b des Behältnisses 10 abdichtet. In dieser Dichtungseinrichtung könnte wiederum eine Öffnung angeordnet sein, über welche Luft aus dem Behältnis 10 abgesaugt werden kann, um im Inneren des Behältnisses 10 ein (Teil-)Vakuum zu erzeugen.

Auch könnte die gesamte Vorrichtung oder auch eine Vielzahl derartiger Vorrichtungen in einem Raum bzw. einem Gehäuse angeordnet sein, welches mit einem (Teil-)Vakuum beaufschlagt wird.

Die oben erwähnte Kühlung des Behandlungskopfes 5 mittels Luft kann sowohl während der eigentlichen Ausstrahlung von Elektronen vorgenommen werden als auch in denjenigen Zeiträumen, in denen keine Bestrahlung vorgenommen wird.

## Patentansprüche

1. Verfahren zum Sterilisieren von Behältnissen (10), wobei ein Behandlungskopf (5), in das Innere eines zu sterilisierenden Behältnisses (10) geführt wird, und der Behandlungskopf (5) im Inneren des Behältnisses (10) während eines vorgegebenen Zeitraums (dT) wenigstens zeitweise Strahlung emittiert, wobei das Behältnis (5) gegenüber dem Behandlungskopf (5) in einer Längsrichtung (L) des Behältnisses (10) während des vorgegebenen zeitraums (dT) wenigstens zeitweise mit einer relativen Bewegungsgeschwindigkeit (v) bewegt wird, wobei diese relative Bewegungsgeschwindigkeit (v) des im inneren des Behältnisses (10) befindlichen Bebandlungskopfes (5) während des vorgegebenen zeitraums (dT) variiert und in Ahängigkeit eines Innenprofils (10a) des Behältnisses (10) gesteuert wird **dadurch gekennzeichnet, dass**
die Bewegung aufgrund eines programmierten Fahrprofils gesteuert wird und das Fahrprofil ermittelt wird, indem ein Behandlungskopf (5) in das Innere eines Behältnisses (10) geführt wird und der Behandlungskopf (5) im Inneren des Behältnisses (10) Strahlung emittiert, wobei das Behältnis (5) gegenüber dem Behandlungskopf (5) während der Emission der Strahlung in einer Längsrichtung (L) des Behältnisses (10) bewegt und eine Vielzahl von charakteristischen Daten aufgenommen werden, welche für eine auf eine Innenwandung des Behältnisses auftreffende Strahlung charakteristisch sind oder die Bewegung aufgrund eines programmierten Fahrprofils gesteuert wird und das Fahrprofil ermittelt wird, indem mittels einer Bildaufnahmeeinrichtung ein Bild eines zu sterilisierenden Behältnisses aufgenommen wird und die Daten dieser Bildaufnahme für die Geschwindigkeitssteuerung verwendet werden.

2. Verfahren nach Anspruch 1, **dadurch gekennzeichnet, dass** der Behandlungskopf (5) Ladungsträger emittiert.

3. verfahren nach wenigstens einem der vorangegangenen Ansprüche, **dadurch gekennzeichnet, dass** die Bewegung durch eine Steuereinrichtung (12) auf Grundlage von in einer Speichereinrichtung (14) abgelegten Daten gesteuert wird, wobei diese Daten für Querschnitte des Behältnisses in der Längsrichtung (L) des Behältnisses (10) charakteristisch sind.

4. verfahren nach wenigstens einem der vorangegangenen Ansprüche, **dadurch gekennzeichnet, dass** die Bewegung des Behandlungskopfes in Ahängigkeit von einem Durchmesser D(L) des Behältnisses (10) gesteuert wird,

5. verfahren nach wenigstens einem der vorangegangenen Ansprüche, **dadurch gekennzeichnet, dass** die Bewegung des Behandlungskopfes (5) in Abhängigkeit von einem Winkel gesteuert wird, unter dem sich die Innenwandung des Behältnisses (10) gegenüber der Längsrichtung (L) des Behältnisses (10) erstreckt.

6. Verfahren nach wenigstens einem der vorangegangenen Ansprüche, **dadurch gekennzeichnet, dass** das Behältnis (10) in seiner Längsrichtung (L) bewegt wird.

7. Verfahren nach Anspruch 6, **dadurch gekennzeichnet, dass** an einer Innenwandung des Behältnisses (10) ein Material ausgebracht wird, welches auf die von dem Behandlungskopf (5) ausgesandte Strahlung reagiert und Rückschlüsse auf eine Intensität dieser Strahlung zulässt.

8. Verfahren zur Bestimmung eines Fahrprofils für die Innensterilisation von Behältnissen, wobei ein Behandlungskopf (5), in das innere eines Behältnisses geführt wird, und der Behandlungskopf (5) im Inneren des Behältnisses (10) Strahlung auf eine Innenwandung (10a) des Behältnisses richtet, wobei das Behältnis (5) gegenüber dem Behandlungskopf (5) während der Emission der Strahlung in einer Längsrichtung (L) des Behältnisses (10) bewegt wird, **dadurch gekennzeichnet, dass** eine Vielzahl von Daten aufgenommen wird, welche für eine auf eine Innenwandung (10a) des Behältnisses (10) auftreffende Strahlung charakteristisch sind.

9. Vorrichtung zum Sterilisieren von Behältnissen (10) mit einem Behandlungskopf (5) der derart gestaltet ist, dass er in einen Innenraum eines zu sterilisierenden Behältnisses (10) führbar ist, wobei der Behandlungskopf (5) eine Strahlungsquelle aufweist, welche Strahlung ausgibt und wobei die Vorrichtung eine Bewegungseinrichtung aufweist, welche das Behältnis gegenüber dem Behandlungskopf in einer Längsrichtung (L) des Behältnisses (10) mit einer vorgegebenen Bewegungsgeschwindigkeit bewegt,
**dadurch gekennzeichnet, dass** die Vorrichtung (1) eine Steuereinrichtung (12) aufweist, welche diese relative Bewegungsgeschwindigkeit des im inneren des Behältnisses (10) befindlichen Behandlungskopfes (5)aufgrund eines gemäß Anspruch 8 bestimmten programmierten Fahrprofils in Abhängigkeit eines Innenprofils des Behältnisses (10) variiert, wobei die Bewegung aufgrund des programmierten Fahrprofils gesteuert wird, wobei die Vorrichtung (1) eine Bildaufnahmeeinrichtung (16) aufweist, welche ein Bild eines zu sterilisierenden Behältnisses (10) aufnimmt, und die Daten dieser Bildaufnahme für die Geschwindigkeitssteuerung verwendet werden, und wobei in einem Arbeitsbetrieb ein Bild eines Behältnisses (10) aufgenommen wird und anschließend eine Bildverarbeitungssoftware dieses Bild mit einer vielzahl von Bildern für abgespeicherte Behältnisse vergleicht, und sobald ein entsprechendes Behältnis gefunden wurde ein entsprechendes Fahrprofil geladen wird und im Arbeitsbetrieb aufgrund dieses Fahrprofils die Bewegung des Behandlungskopfes gesteuert wird, oder das Fahrprofil wird sowohl aufgrund von direkten geometrischen Überlegungen als auch aufgrund von empirischen, gemäß Anspruch 8 bestimmten, Daten gesteuert.

10. vorrichtung nach Anspruch 9, **dadurch gekennzeichnet, dass** die Strahlungsquelle Ladungsträger und insbesondere Elektronen aussendet.

11. Vorrichtung (1) nach wenigstens einem der vorangegangenen Ansprüche 9 bis 10, **dadurch gekennzeichnet, dass** die Vorrichtung (1) eine Speichereinrichtung (14) aufweist, in der ein für die Bewegung des Behandlungskopfes charakteristisches Fahrprofil gespeichert ist.

12. Vorrichtung (1) nach wenigstens einem der vorangegangenen Ansprüche 9 bis 11, **dadurch gekennzeichnet, dass** die vorrichtung eine Vielzahl von Behandlungsköpfen (5) aufweist, welche hintereinander an einer Transporteinrichtung (31) angeordnet sind.

## Claims

1. Method for sterilising containers (10), wherein a treatment head (5) is guided into the interior of a container (10) to be sterilised and the treatment head (5) emits radiation in the interior of the container (10) at least intermittently over a predefined time period (dT), wherein the container (5) is moved relative to the treatment head (5) in a longitudinal direction (L) of the container (10) at least intermittently at a relative movement speed (v) over the predefined time period (dT), wherein this relative movement speed (v) of the treatment head (5) located in the interior of the container (10) varies over the predefined time period (dT) and is controlled as a function of an internal profile (10a) of the container (10), **characterized in that** the movement due to a programmed travel profile is controlled and the travel profile is determined by guiding the treatment head (5) in the interior of a container and by the fact that the treatment head (5) emits radiation in the interior of the container (10), wherein the container (5) is moved with respect to the treatment head (5) during the emission of the radiation in a longitudinal direction (L) of the container (10) and a plurality of characteristic data are received, which are characteristic of radiation impinging an inner wall of the container or the movement is controlled due to a programmed travel profile and the travel profile is detected by means of an image receiving device receiving an image of the container to be sterilized and the data of this image receiving step is used for the speed control.

2. Method according to claim 1, **characterised in that** the treatment head (5) emits charge carriers.

3. Method according to at least one of the preceding claims, **characterised in that** the movement is controlled by a control device (12) on the basis of data stored in a memory device (14), wherein these data are characteristic of cross-sections of the container in the longitudinal direction (L) of the container (10).

4. Method according to at least one of the preceding claims, **characterised in that** the movement of the treatment head is controlled as a function of a diameter D(L) of the container (10).

5. Method according to at least one of the preceding claims, **characterised in that** the movement of the treatment head (5) is controlled as a function of an angle at which the internal wall of the container (10) extends relative to the longitudinal direction (L) of the container (10).

6. Method according to at least one of the preceding claims, **characterised in that** the container (10) is moved in its longitudinal direction (L).

7. Method according to claim 6, **characterised in that** a material which reacts to the radiation emitted by the treatment head (5) and which allows conclusions to be drawn about an intensity of this radiation is applied to an internal wall of the container (10).

8. Method for determining a travel profile for the internal sterilisation of containers, wherein a treatment head (5) is guided into the interior of a container and the treatment head (5) in the interior of the container (10) directs radiation onto an internal wall (10a) of the container, wherein the container (5) is moved relative to the treatment head (5) in a longitudinal direction (L) of the container (10) during the emission of the radiation, **characterised in that** a plurality of data are recorded which are characteristic of radiation impinging an internal wall (10a) of the container (10).

9. Apparatus for sterilising containers (10), comprising a treatment head (5) which is configured in such a way that it can be guided into an interior of a container (10) to be sterilised, wherein the treatment head (5) comprises a radiation source which emits radiation, and wherein the apparatus comprises a movement device which moves the container relative to the treatment head in a longitudinal direction (L) of the container (10) at a predefined movement speed, **characterised in that** the apparatus (1) comprises a control device (12) which varies this relative movement speed of the treatment head (5) located in the interior of the container (10) due to a specific programmed travel profile according to claim 8 as a function of an inner profile of the container (10), wherein the movement is controlled due to the programmed travel profile, wherein the apparatus comprises an image receiving device (16) which receives an image of a container (10) to be sterilized and the data of this image receiving step are used for the speed control and wherein in an operation mode an image of a container (10) is received and subsequently an image processing software compares this image with a plurality of images for stored containers and wherein as soon as a corresponding container has been found a corresponding travel profile is loaded and the movement of the treatment head is controlled in the operation mode due to this travel profile or the travel profile is controlled both due to direct geometric considerations and due to empiric data determined according to claim 8.

10. Apparatus according to claim 9, **characterised in that** the radiation source emits charge carriers and in particular electrons.

11. Apparatus (1) according to at least one of the preceding claims 9 to 10, **characterised in that** the apparatus (1) comprises a memory device (14), in which a travel profile characteristic of the movement of the treatment head is stored.

12. Apparatus (1) according to at least one of the preceding claims 9 to 11, **characterised in that** the apparatus comprises a plurality of treatment heads (5) which are arranged one behind the other on a transport device (31).

## Revendications

1. Procédé de stérilisation de récipients (10), dans lequel une tête de traitement (5) est introduite à l'intérieur d'un récipient (10) à stériliser, et dans le quel la tête de traitement (5) émet un rayonnement à l'intérieur du récipient (10) au moins temporairement pendant une durée (dT) définie, le récipient (10) étant déplacé au moins temporairement pendant la durée (dT) définie avec une vitesse de déplacement (v) relative par rapport à la tête de traitement (5) dans une direction longitudinale (L) du récipient (10), ladite vitesse de déplacement (v) relative de la tête de traitement (5) introduite à l'intérieur du récipient (10) variant pendant la durée (dT) définie et étant commandée en fonction d'un profil intérieur (10a) du récipient (10),
**caractérisé en ce que**
le déplacement est commandé sur la base d'un profil de déplacement programmé et **en ce que** le profil de déplacement est déterminé par introduction d'une tête de traitement (5) à l'intérieur d'un récipient (10) et par émission de rayonnement par la tête de traitement (5) à l'intérieur du récipient (10), le récipient (5) étant déplacé par rapport à la tête de traitement (5) dans une direction longitudinale (L) du récipient (10) pendant l'émission du rayonnement et une pluralité de données caractéristiques étant enregistrée, lesquelles sont caractéristiques pour un rayonnement incident sur une paroi intérieure du récipient, ou **en ce que** le déplacement est commandé sur la base d'un profil de déplacement programmé et le profil de déplacement est déterminé par enregistrement d'une image d'un récipient à stériliser au moyen d'un dispositif d'enregistrement d'image, les données de cet enregistrement d'image étant exploitées pour la commande de vitesse.

2. Procédé selon la revendication 1, **caractérisé en ce que** la tête de traitement (5) émet des porteurs de charge.

3. Procédé selon au moins une des revendications précédentes, **caractérisé en ce que** le déplacement est commandé par un dispositif de commande (12) sur la base de données stockées dans un dispositif de mémorisation (14), lesdites données étant caractéristiques pour des sections transversales du récipient dans la direction longitudinale (L) du récipient (10).

4. Procédé selon au moins une des revendications précédentes, **caractérisé en ce que** le déplacement de la tête de traitement est commandé en fonction d'un diamètre D(L) du récipient (10).

5. Procédé selon au moins une des revendications précédentes, **caractérisé en ce que** le déplacement de la tête de traitement (5) est commandé en fonction d'un angle suivant lequel la paroi intérieure du récipient (10) s'étend par rapport à la direction longitudinale (L) du récipient (10).

6. Procédé selon au moins une des revendications précédentes, **caractérisé en ce que** le récipient (10) est déplacé dans sa direction longitudinale (L).

7. Procédé selon la revendication 6, **caractérisé en ce qu'**un matériau est appliqué sur une paroi intérieure du récipient (10), lequel réagit au rayonnement émis par la tête de traitement (5) et permet de déduire une intensité dudit rayonnement.

8. Procédé de détermination d'un profil de déplacement pour la stérilisation intérieure de récipients, dans lequel une tête de traitement (5) est introduite à l'intérieur d'un récipient à stériliser, et la tête de traitement (5) dirige à l'intérieur du récipient (10) un rayonnement vers une paroi intérieure (10a) du récipient, le récipient (10) étant déplacé par rapport à la tête de traitement (5) dans une direction longitudinale (L) du récipient (10) pendant l'émission du rayonnement, **caractérisé en ce qu'**une pluralité de données est enregistrée, lesquelles sont caractéristiques pour un rayonnement incident sur une paroi intérieure (10a) du récipient (10).

9. Installation de stérilisation de récipients (10) avec une tête de traitement (5) réalisée de manière à pouvoir être introduite à l'intérieur d'un récipient (10) à stériliser, la tête de traitement (5) comportant une source de rayonnement émettant un rayonnement et l'installation comportant un dispositif de déplacement qui déplace le récipient avec une vitesse de déplacement définie par rapport à la tête de traitement dans une direction longitudinale (L) du récipient (10),
**caractérisée en ce que**
ladite installation (1) comporte un dispositif de commande (12) qui varie ladite vitesse de déplacement relative de la tête de traitement (5) introduite à l'intérieur du récipient (10) en fonction d'un profil intérieur du récipient (10) sur la base d'un profil de déplacement programmé déterminé selon la revendication 8, le déplacement étant commandé sur la base du profil de déplacement programmé, l'installation (1) comportant un dispositif d'enregistrement d'image (16) qui enregistre une image d'un récipient (10) à stériliser, les données de cet enregistrement d'image étant exploitées pour la commande de vitesse, et une image d'un récipient (10) étant enregistrée en mode de travail, ladite image étant ensuite comparée par un logiciel de traitement d'image à une pluralité d'images pour des récipients mémorisés, un profil de déplacement correspondant étant chargé dès qu'un récipient correspondant est trouvé, et le déplacement de la tête de traitement étant commandé en mode de travail sur la base dudit profil de déplacement, ou le profil de déplacement étant commandé aussi bien sur la base de réflexions géométriques directes que sur celle de données empiriques déterminées selon la revendication 8.

10. Installation selon la revendication 9, **caractérisé en ce que** la source de rayonnement émet des porteurs de charge et en particulier des électrons.

11. Installation (1) selon au moins une des revendications 9 à 10, **caractérisé en ce que** ladite installation (1) comporte un dispositif de mémorisation (14) dans lequel est mémorisé un profil de déplacement caractéristique pour le déplacement de la tête de traitement.

12. Installation (1) selon au moins une des revendications 9 à 11, **caractérisé en ce que** ladite installation comporte une pluralité de têtes de traitement (5) disposées les unes derrières les autres sur un dispositif de transport (31).
